Europäisches Patentamt

(19) European Patent Office

Office européen des brevets

(11) Numéro de publication: **0 100 114**
**B1**

(12) **FASCICULE DE BREVET EUROPEEN**

(45) Date de publication du fascicule du brevet: **20.11.86**

(51) Int. Cl.⁴: **A 61 B 17/58**

(21) Numéro de dépôt: **83200989.8**

(22) Date de dépôt: **04.07.83**

(54) **Plaques d'ostéosynthèse pour dérotation osseuse.**

(30) Priorité: **26.07.82 FR 8213148**

(43) Date de publication de la demande:
**08.02.84 Bulletin 84/06**

(45) Mention de la délivrance du brevet:
**20.11.86 Bulletin 86/47**

(84) Etats contractants désignés:
**BE CH DE GB IT LI LU NL**

(56) Documents cités:
**FR-A-1 051 847**
**FR-A-1 529 298**
**US-A-3 463 148**

(73) Titulaire: **INSTITUT NATIONAL DE LA SANTE ET DE LA RECHERCHE MEDICALE (INSERM)**
**101, rue de Tolbiac**
**F-75654 Paris Cedex 13 (FR)**

(72) Inventeur: **Darmana, Robert**
**4 bis, rue Averseng Delorme**
**F-31000 Toulouse (FR)**
Inventeur: **Laville, Jean-Marc**
**29 allées F. Verdier**
**F-31000 Toulouse (FR)**
Inventeur: **Morucci, Jean-Pierre**
**75 rue du Cagire**
**F-31100 Toulouse (FR)**
Inventeur: **Nabonne, Patrick**
**Place Mahomme**
**F-32190 Vic Fezensac (FR)**
Inventeur: **Pasquie, Maurice**
**21 boulevard Carnot**
**F-31000 Toulouse (FR)**

(74) Mandataire: **Barre, Philippe**
**Cabinet Barre, Gatti, Laforgue 95, rue des Amidonniers**
**F-31069 Toulouse Cedex (FR)**

Courier Press, Leamington Spa, England.

EP 0 100 114 B1

## Description

L'invention concerne une plaque d'ostéosynthèse pour dérotation osseuse, destinée à positionner et assujettir l'un par rapport à l'autre, dans une position angulaire prédéterminée autour de leur axe longitudinal, deux segments d'un os (en particulier d'un tibia), obtenus après une coupe transversale de cet os.

Un pourcentage notable d'enfants et certains adultes possèdent des anomalies rotationnelles des membres inférieurs, qui, outre leur caractère inesthétique, impliquent un grave danger de détérioration, à plus ou moins long terme, du cartilage rotulien sur lequel se concentrent les efforts. Pour remédier à cette malformation, les chirurgiens sont souvent amenés à pratiquer une ostéotomie tibiale, qui consiste à sectionner transversalement le tibia du membre considéré, à faire pivoter la partie inférieure, porteuse du pied, par rapport à la partie supérieure, d'un angle, dit de dérotation, qui permettra d'obtenir un positionnement correct du pied, puis à mettre en place une plaque d'ostéosynthèse pour maintenir les deux segments de l'os sectionné dans la bonne position, le temps de la régénération osseuse.

Actuellement, dans les dérotations du tibia (dont la section transversale est à peu près triangulaire), les chirurgiens utilisent des plaques rectilignes, qu'ils vrillent au moyen de deux pinces avant leur mise en place, afin de créer une torsade adaptée à l'angle de dérotation désiré; la plaque ainsi vrillée est vissée par ses extrémités sur la face interne de chaque segment du tibia afin de maintenir ces segments avec le décalage angulaire recherché.

Toutefois ce type de plaque présente les graves inconvénients suivants. En premier lieu la plaque ainsi vrillée ne permet pas d'obtenir un positionnement correct des segments osseux; en effet, en raison de la présence de la torsade, ce type de plaque possède le double défaut, lorsqu'elle est vissée sur les segments osseux, d'une part, de tendre à ramener les segments vers une position de moindre décalage angulaire, d'autre part, d'éloigner les segments osseux l'un de l'autre et d'écarter leur coupe; il est ainsi à peu près impossible avec de telles plaques d'obtenir une dérotation donnée, un alignement précis des segments, et des coupes en contact (en vue de permettre à la régénération osseuse de s'effectuer dans de bonnes conditions).

De plus de vrillage de la plaque en bloc opératoire est une opération délicate et peu pratique, qui conduit à une grande imprécision dans l'exécution de la torsade, implique des pertes de temps notables et requiert un effort musculaire relativement important de la part du chirurgien.

La présente invention se propose de palier les inconvénients sus-évoqués de la technique antérieure, en fournissant de nouvelles plaques d'ostéosynthèse adaptées à une dérotation tibiale et, plus généralement à toute dérotation dans laquelle l'os concerné ne présente pas une section circulaire.

Un objectif essentiel de l'invention est en particulier de permettre l'obtention d'une dérotation d'un angle précis prédéterminé sans modification de cet angle au cours de la mise en place de la plaque.

Un autre objectif de l'invention est de garantir une application des segments osseux l'un contre l'autre au niveau de leur coupe.

Un autre objectif est de réaliser la dérotation avec un alignement précis des axes longitudinaux des deux segments.

Un autre objectif est de permettre une amélioration de la régénération osseuse au voisinage de la coupe des segments osseux.

Un autre objectif est de supprimer toute opération de mise en forme de la plaque en bloc opératoire.

A cet effet, la plaque d'ostéosynthèse visée par l'invention comprend deux faces d'appui dotées chacune de moyens de fixation sur un segment osseux; conformément à la présente invention, dans la portion centrale de la plaque, ces deux faces d'appui sont interrompues par des décrochements et s'étendent de part et d'autre desdits décrochements dans deux plans formant entre eux un angle prédéterminé correspondant à l'angle de dérotation.

Selon une autre caractéristique de l'invention, la plaque comprend, entre ses faces d'appui, un tronçon central, situé entre les deux décrochements précités et formant un évidement tel que tous les points dudit tronçon se trouvent en retrait par rapport à chacune des faces d'appui.

Comme on le comprendra mieux plus loin, le terme "retrait" ci-dessus utilisé se réfère au corps de la plaque et signifie qu'aucun point du tronçon central ne se trouve en saillie du côté opposé au corps, par rapport aux faces d'appui.

De plus les faces d'appui peuvent ne pas être rigoureusement planes et les plans précités selon lesquels elles s'étendent, doivent être interprétés comme des plans moyens, sans obligation de coïncidence en tout point.

Selon une autre caractéristique de l'invention, les faces d'appui sont agencées l'une dans le prolongement de l'autre de façon que leur axe médian longitudinal coïncide, les deux plans contenant lesdites faces se coupant selon cet axe médian et étant angulairement décalés autour de celui-ci de l'angle précité.

Ainsi la plaque d'ostéosynthèse n'est pas façonnée sur place et ne comporte plus de torsade; une de ses faces d'appui est fixée sur un des segments osseux de façon que la coupe de ce dernier vienne se situe au niveau du tronçon central évidé de la plaque; après rotation, la coupe de l'autre segment osseux est appliquée contre la coupe du premier segment et la seconde face d'appui de la plaque est fixée sur cet autre segment. On obtient ainsi une fixation rigide des deux segments dans l'alignement l'un de l'autre, avec un angle de dérotation précis égal à l'angle que forment les deux faces d'appui.

La plaque d'ostéosynthèse conforme à l'invetion peut, notamment, être réalisée par usinage à partir d'un barreau en un matériau biocompatible.

L'invention s'étend à un jeu de plaques d'ostéosynthèse dont les faces d'appui sont situées dans des plans formant des angles différents d'une plaque à l'autre; les angles des différentes plaques peuvent par exemple être répartis de 5° en 5° dans une plage allant de 10° à 40°, le chirurgien choisissant dans chaque cas la plaque la plus appropriée.

L'invention sera mieux comprise à la lecture de la description qui suit en référence aux dessins annexés, qui en présentent à titre non limitatif un mode de réalisation préférentiel; sur ces dessins qui font partie intégrante de la présente description:

— la figure 1 est une vue en perspective, à échelle dilatée, d'une plaque d'ostéosynthèse conforme à l'invention,
— la figure 2 est une vue de côte de ladite plaque,
— les figures 3 et 4 en sont des coupes transversales, respectivement par des plans CC et AA,
— la figure 5 est une vue schématique, en perspective, montrant la plaque fixée sur les deux segments d'un tibia,
— la figure 6 est une vue frontale selon V de cette plaque ainsi mise en place, cependant que la figure 7 en est un coupe par un plan DD.

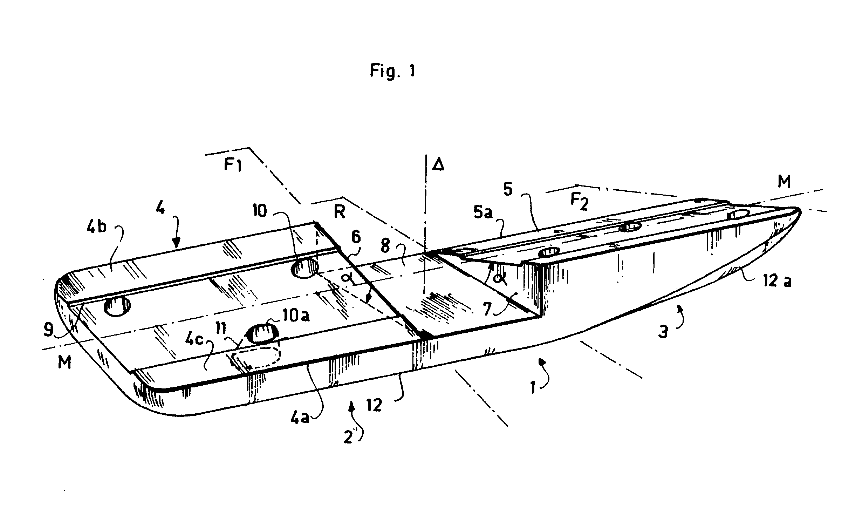

La plaque d'ostéosynthèse représentée à titre d'exemple aux figures 1, 2, 3 et 4 est destinée à permettre la dérotation d'un tibia (ou de tout autre os de section, non circulaire, présentant au moins une face à peu près plane). Dans le cas d'un tibia, ladite plaque est appelée à se fixer, après coupe transversale du tibia, sur la face interne des deux segments de celui-ci.

Ladite plaque est réalisée par usinage sur machine-outil à partir d'un barreau, ou éventuellement par moulage, en une matière biocompatible, telle que acier inoxydable, résine synthétique biocompatible etc...

Elle comprend essentiellement sur sa longueur trois tronçons: un tronçon central 1 et deux tronçons 2 et 3 situés de part et d'autre du tronçon central.

Les tronçons 2 et 3 comportent des faces d'appui 4 et 5 qui sont interrompues au niveau du tronçon central, par des décrochements 6 et 7 formant deux facettes qui bordent latéralement le tronçon central.

Les faces d'appui 4 et 5 sont agencées de sorte que leur axe médian M coïncide; elles sont contenues dans deux plans $F_1$ et $F_2$ qui se coupent selon cet axe médian et forment entre eux un angle $2\alpha$ prédéterminé.

Le tronçon central 1 qui est bordé par les facettes 6 et 7 forme un évidement possédant un fond 8 dont tous les points sont situés en retrait ou à fleur par rapport à un plan de référence R passant par les deux arêtes longitudinales opposées 4a et 5a des faces d'appui 4 et 5 (ces arêtes étant celles qui se trouvent les plus en retrait vers la surface externe 12 de la plaque).

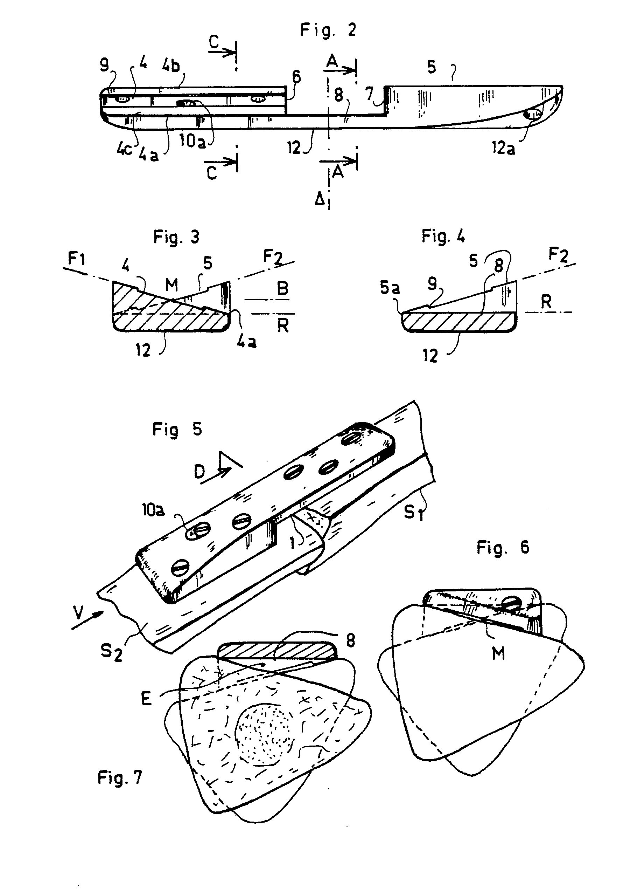

En l'exemple les facettes 6 et 7 sont identiques et de forme triangulaire et sont symétriques l'une de l'autre par rapport à un axe central $\Delta$ perpendiculaire au plan de référence R; les angles de ces facettes (qui correspondent aux sommets situés sur les arêtes 4a et 5a), sont chacun égaux à $\alpha$, moitié de l'angle de dérotation $2\alpha$ que forment les faces d'appui (le plan de référence R qui passe par les arêtes 4a et 5a, étant parallèle au plan bissecteur B des deux faces comme le montre la figure 3). De plus le fond 8 est en l'exemple plan et est situé dans ce plan de référence R, sa jonction avec les facettes 6 et 7 s'effectuant par les deux arêtes transversales inférieures desdites facettes.

La dimension longitudinale du tronçon central (distance entre les deux facettes 6 et 7) est approximativement comprise entre 10 et 30 mm, de l'ordre de 20 mm par exemple, cependant que la longueur des faces d'appui peut être de l'ordre de 40 à 50 mm.

Par ailleurs chaque face d'appui 4 ou 5 présente autour de son axe médian, une rainure longitudinale, définie par deux petits épaulements 9 et qui délimite en bordure de ladite face d'appui deux bandes longitudinales telles que 4b et 4c formant la surface d'appui de ladite face. Cette surface est ainsi adaptée pour s'appuyer stablement sur la face interne du tibia malgré le caractère légèrement convexe de celle-ci.

De plus la plaque d'ostéosynthèse est dotée de moyens de fixation constitués par des vis (non représentées aux figures 1 à 4) appelées à s'engager dans des perçages tels que 10, en l'exemple au nombre de six, répartis selon une disposition triangulaire sur les faces d'appui.

L'un de ces perçages 10a présente une forme oblongue et possède une portée inclinée 11 du côté opposé au tronçon central. De la sorte, en fin de vissage, la tête de vis engagée dans ce perçage tend à glisser sur cette portée vers les coupes des segments osseux et à rapprocher le segment dans lequel elle est vissée, de l'autre segment; cette vis d'autocompression garantit ainsi un appui des deux segments par leur coupe.

Par ailleurs la face externe 12 de la plaque est de préférence à peu près parallèle au plan de référence R dans sa partie centrale, avec un arrondi 12a à chaque extrémité afin de limiter l'épaisseur de la plaque à ce niveau, d'alléger celle-ci et de ne pas léser les tissus avoisinants.

Les figures 5, 6 et 7 illustrent la mise en place d'un plaque d'ostéosynthèse et permettent d'en saisir les divers avantages.

Le chirurgien dispose d'un jeu de plaques dont les angles de dérotation $2\alpha$ varient entre 10° et 40°, en particulier de 5° en 5°, ce qui permet en pratique de satisfaire avec précision tous les besoins.

Le tibia est préalablement sectionné et la plaque est vissée sur le segment supérieur $S_1$ de façon que la coupe d'extrémité de celui-ci vienne se situer au niveau du tronçon central 1 de la plaque.

L'autre segment S₂, porteur du pied, est amené à pivoter de l'angle de dérotation 2α et sa coupe est appliquée contre celle du premier segment. L'autre face d'appui de la plaque est à son tour vissée sur le second segment S₂, en commençant par la vis qui est engagée dans le perçage 10a à portée inclinée; le vissage de cette vis met en compression les coupes des deux segments l'une contre l'autre.

Il est à noter que, comme l'illustre la figure 7, la plaque conditionne la présence d'une partie vide E entre elle-même et les segments osseux, au niveau de son tronçon central; l'os garde ainsi la faculté de se regénérer à ce niveau pour reformer peu à peu la continuité entre les deux segments.

On conçoit l'intérêt des plaques d'ostéosynthèse conformes à l'invention, qui permettent de réaliser une dérotation d'angle précis invariable prédéterminé, de conserver alignés les axes longitudinaux des segments (passant par le barycentre de leur section triangulaire) et d'améliorer les conditions de régénération osseuse tout en supprimant toute opération de mise en forme en bloc opératoire.

## Revendications

1. Plaque d'ostéosynthèse pour dérotation osseuse, destinée à positionner et assujettir l'un par rapport à l'autre deux segments osseux, ladite plaque comprenant deux faces d'appui (4, 5) dotées chacune de moyens de fixation (10) sur un segment osseux, et étant caractérisée en ce que, dans la partie centrale de la plaque, les deux faces d'appui (4, 5) sont interrompues par des décrochements (6, 7) et s'étendent de part et d'autre desdits décrochements dans deux plans (F₁, F₂) formant entre eux un angle (2α) prédéterminé correspondant à l'angle de dérotation.

2. Plaque d'ostéosynthèse selon la revendication 1, caractérisée en ce qu'elle comprend, entre ses faces d'appui (4, 5) un tronçon central (1), situé entre les deux décrochements (6, 7) précités et formant un évidement tel que tous les points dudit tronçon se trouvent en retrait par rapport à chacune des faces d'appui (4, 5).

3. Plaque d'ostéosynthèse selon l'une des revendications 1 ou 2, caractérisée en ce que ses faces d'appui (4, 5) sont agencées l'une dans le prolongement de l'autre de façon que leur axe médian longitudinal (M) coïncide, les deux plans (F₁, F₂) contenant lesdites faces se coupant selon cet axe médian et étant angulairement décalés autour de celui-ci de l'angle (2α) précité.

4. Plaque d'ostéosynthèse selon les revendications 2 et 3 prises ensemble, caractérisée en ce que le tronçon central (1) formant évidement est bordé de deux facettes latérales formées par les deux décrochements (6, 7) précités et comprend un fond (8) situé en retrait ou à fleur par rapport à un plan de référence (R) passant par les deux arêtes longitudinales opposées (4a, 5a) des deux faces d'appui, (arêtes situées les plus en retrait).

5. Plaque d'ostéosynthèse selon la revendication 4, caractérisée en ce que les deux facettes (6, 7) sont identiques et symétriques l'une de l'autre par rapport à un axe central (Δ) perpendiculaire au plan de référence (R) et présentent une forme triangulaire dotée de deux arêtes transversales de jonction avec le fond.

6. Plaque d'ostéosynthèse selon l'une des revendications 4 ou 5, caractérisée en ce que le tronçon central (1) formant évidement présente une dimension longitudinale approximativement comprise entre 10 et 30 mm.

7. Plaque d'ostéosynthèse selon l'une des revendications 1, 2, 3, 4, 5 ou 6, caractérisée en ce que chaque face d'appui (4, 5) présente une rainure longitudinale délimitant en bordure de ladite face d'appui deux bandes longitudinales (4b, 4c) formant la surface d'appui de ladite face (4, 5).

8. Plaque d'ostéosynthèse selon l'une des revendications précédentes, dans laquelle les moyens de fixation sont constitués par des vis engagées dans des perçages (10) de la plaque, l'un desdits perçages (10a) étant de forme oblongue et possédant une portée inclinée (11) du côté opposé au tronçon central (1).

9. Jeu de plaques d'ostéosynthèse conformes à l'une des revendications précédentes et présentant des faces d'appui (4, 5) situés dans des plans (F₁, F₂) formant des angles (2α) différents d'une plaque à l'autre.

10. Jeu de plaques d'ostéosynthèse selon la revendication 9, caractérisé en ce que les angles (2α) des différentes plaques sont régulièrement répartis dans une plage allant de 10° à 40°.

## Patentansprüche

1. Osteosyntheseplatte für die Knochenderotation zur Positionierung und Verbindung von zwei Knochensegmenten; diese Platte besitzt zwei mit Mitteln (10) zur Befestigung an einem Knochensegment versehene Auflageflächen (4, 5) und ist dadurch gekennzeichnet, daß die beiden Auflageflächen (4, 5) im mittleren Bereich der Platte von Absätzen (6, 7) unterbrochen werden und beiderseits dieser Absätze in zwei Ebenen (F₁, F₂) verlaufen, die in einem festgelegten Winkel (2α) zueinander stehen, der dem Derotationswinkel entspricht.

2. Osteosyntheseplatte gemäß Anspruch 1, dadurch gekennzeichnet, daß sich zwischen den Auflageflächen (4, 5) eine zwischen den beiden vorgenannten Absätzen (6, 7) liegende Mittelsektion (1) befindet, die eine Aussparung bildet, so daß alle Punkte dieser Mittelsektion tiefer liegen als die beiden Auflageflächen (4, 5).

3. Osteosyntheseplatte gemäß einem der Ansprüche 1 oder 2, dadurch gekennzeichnet, daß ihre Auflageflächen (4, 5) so hintereinander angeordnet sind, daß ihre mittlere Längsachse übereinstimmt, die beiden diese Flächen beinhaltenden Ebenen (F₁, F₂) sich in dieser Mittelachse schneiden und um diese Achse herum um

den vorgenannten Winkel (2α) zueinander versetzt sind.

4. Osteosyntheseplatte gemäß den vereinten Ansprüchen 2 und 3, dadurch gekennzeichnet, daß die eine Aussparung bildende Mittelsektion (1) von zwei durch die beiden vorgenannten Absätze (6, 7) gebildeten Seitenflächen begrenzt wird und einen Boden (8) besitzt, der im Verhältnis zu einer durch die beiden entgegengesetzten Längskanten (4a, 5a) (die am tiefsten liegenden Kanten) der beiden Auflageflächen verlaufenden Bezugsebene (R) tiefer oder bündig liegt.

5. Osteosyntheseplatte gemäß Anspruch 4, dadurch gekennzeichnet, daß die beiden Seitenflächen (6, 7) identisch und im Verhältnis zu einer senkrecht zur Bezugsebene (R) stehenden Mittelachse (Δ) miteinander symmetrisch sind.

6. Osteosyntheseplatte gemäß einem der Ansprüche 4 oder 5, dadurch gekennzeichnet, daß die eine Aussparung bildende Mittelsektion (1) etwa zwischen 10 und 30 mm lang ist.

7. Osteosyntheseplatte gemäß einem der Ansprüche 1, 2, 3, 4, 5 oder 6, dadurch gekennzeichnet, daß jede Auflagefläche (4, 5) der Länge nach mit einer Einkerbung versehen ist, die an den Rändern der Auflagefläche zwei Längsleisten (4b, 4c) begrenzt, welche den tatsächlich aufliegenden Bereich dieser Auflagefläche (4, 5) bilden.

8. Osteosyntheseplatte gemäß einem der vorstehenden Ansprüche, bei der die Mittel zur Befestigung an den Knochensegmenten aus in die Bohrungen (10) der Platte eingesetzten Schrauben bestehen, wobei eine dieser Bohrungen (10a) eine längliche Form hat und auf der Mittelsektion (1) gegenüberliegenden Seite eine abgeschrägte Auflagefläche (11) besitzt.

9. Satz von Osteosyntheseplatten gemäß einem der vorstehenden Ansprüche, deren Auflageflächen (4, 5) in Ebenen (F₁, F₂) liegen, deren Winkel zueinander (2α) von einer Platte zur anderen verschieden ist.

10. Satz von Osteosyntheseplatten gemäß Anspruch 9, dadurch gekennzeichnet, daß die Winkel (2α) der verschiedenen Platten regelmäßige Abstände voneinander haben und von 10° bis 40° reichen.

## Claims

1. Osteosynthetic plate for bone derotation, designed to position and secure two bone segments one in relation to the other, the said plate comprising two bearing surfaces (4, 5) each provided with means of attachment (10) to a bone segment, wherein, in the central part of the plate, the two bearing surfaces (4, 5) are interrupted by steps (6, 7) and extend either side of the said steps on two planes (F₁, F₂) forming together a predetermined angle (2α) corresponding to the angle of derotation.

2. Osteosynthetic plate in accordance with claim 1, characterized by comprising, between its bearing surfaces (4, 5) a central section (1), located between the two aforementioned steps and forming a recess such that all the points on the said section are sunken with respect to each of the bearing surfaces (4, 5).

3. Osteosynthetic plate in accordance with one of the claims 1 or 2, characterized by the fact that its bearing surfaces (4, 5) are disposed one as the extension of the other so that their longitudinal central axis (M) coincides, the two planes (F₁, F₂) containing the said surfaces intersecting each other on this central axis and being angularly offset around the latter by the aforementioned angle (2α).

4. Osteosynthetic plate in accordance with claims 2 and 3 combined, characterized by the fact that the central section (1) forming a recess is delimited by two lateral surfaces formed by the two aforementioned steps (6, 7) and comprises a bottom surface (8) located sunken or flush with respect to the reference plane (R) passing through the two opposite longitudinal edges (4a, 5a) of the two bearing surfaces (the most sunken edges).

5. Osteosynthetic plate in accordance with claim 4, characterized by the fact that the two lateral surfaces (6, 7) are identical and symmetrical to each other with respect to a central axis (Δ) perpendicular to the reference plane (R) and having a triangular form provided with two transversal edges joining the bottom.

6. Osteosynthetic plate in accordance with one of the claims 4 or 5, characterized by the fact that the central section (1) forming a recess has a longitudinal dimension approximately comprised between 10 and 30 mm.

7. Osteosynthetic plate in accordance with one of the claims 1, 2, 3, 4, 5 or 6, characterized by the fact that each bearing surface (4, 5) is provided with a longitudinal groove delimiting at the edges of the said bearing surface two longitudinal reserves (4b, 4c) forming the bearing surface of the said surface (4, 5).

8. Osteosynthetic plate in accordance with one of the foregoing claims, wherein the attachment means consist of screws inserted in the borings (10) in the plate, one of the said borings (10a) being oblong in shape and having a divergent throat (11) on the side opposite the central section (1).

9. Set of osteosynthetic plates in accordance with one of the foregoing claims and having bearing surfaces (4, 5) located within the planes (F₁, F₂) forming angles (2α) different from one plate to the other.

10. Set of osteosynthetic plates in accordance with claim 9, characterized by the fact that the angles (2α) of the various plates are regularly distributed over a range of 10° to 40°.

Fig. 1

0 100 114

Fig. 2

Fig. 3

Fig. 4

Fig 5

Fig. 6

Fig. 7